# EUROPÄISCHE PATENTANMELDUNG

(11) **EP 1 304 644 A2**
(43) Veröffentlichungstag der Anmeldung: **23.04.2003**
(21) Anmeldenummer: 02020663.7
(22) Anmeldetag: 13.09.2002
(51) Int. Cl.: G06F 19/00

(54) **System zur Überprüfung von Behandlungsplänen**

(30) Priorität: 26.09.2001 DE 10147471
(71) Anmelder: SIEMENS AKTIENGESELLSCHAFT, 80333 München (DE)
(72) Erfinder: Abraham-Fuchs, Klaus, 91058 Erlangen (DE); Rumpel, Eva, Dr., 91052 Erlangen (DE)

(57) **Zusammenfassung**

System zur Überprüfung von Behandlungsplänen, insbesondere im Zuge von Disease Management Services zur Patientenaufklärung, -weiterbildung und -motivation, gegebenenfalls kombiniert mit Telemonitoring kritischer Körperwerte und daraus abgeleiteter frühzeitiger Erkennung von Risikosituationen, mit einem Expertensystem mit in einer Computerdatenbank gespeicherten Expertenregeln, das unter Zugriff auf Patientendaten in unterschiedlichen Patientenakten und/oder bei unterschiedlichen Ärzten oder Kliniken selbstständig den Behandlungsplan einer Plausibilitätskontrolle unterzieht, insbesondere wobei das Expertensystem den Behandlungsplan auf Kontraindikationen mit älteren Patientendaten überprüft.

## Beschreibung

Die Erfindung bezieht sich auf ein System zur Überprüfung von Behandlungsplänen, insbesondere im Zuge von Disease Management Services zur Patientenaufklärung, -weiterbildung, und - motivation, gegebenenfalls kombiniert mit Telemonitoring kritischer Körperwerte und daraus abgeleiteter frühzeitiger Erkennung von Risikosituationen.

Disease Management Services betreuen typischerweise Patienten, die an einer chronischen Volkskrankheit leiden, wie z. B. Diabetes, Asthma oder Hypertonie. Damit werden große Patientenzahlen mit einem weitgehend standardisierten Behandlungsplan über lange Zeiträume (typisch Monate oder Jahre) betreut, was zu einer deutlich gesteigerten Kosteneffizienz gegenüber traditioneller Patientenversorgung führt. Die Standardisierung der Behandlungspläne erreicht jedoch Grenzen, da auf die individuelle Situation jedes Patienten eingegangen werden muss, die sich aber aufgrund ihres Alters, des Schweregrades der Erkrankung, einer eventuell vorhandenen Medikamenten-Unverträglichkeit oder Multimorbidität voneinander unterscheiden. Daher müssen die standardisierten Behandlungspläne individuell angepasst und gegebenenfalls im Laufe des Betreuungszeitraums verändert werden.

Bisher werden die Behandlungspläne durch den medizinisch geschulten Experten individuell erstellt und überwacht, was aber angesichts der hohen Kosten, die angestrebte Kosteneffizienz von Disease Management Services gegenüber traditioneller Patientenversorgung teilweise wieder aufhebt.

Aus der US 5,517,405 ist zwar bereits ein Expertensystem vorgeschlagen worden, um eine interaktive Unterstützung des Arztes bei der Erstellung eines Behandlungsplans zu unterstützen, bei dem aber durch ein mehr oder weniger geführtes Frage-Antwort-Verfahren das Expertensystem die als Vorschlag für eine bestimmte Krankheit einzusetzende Behandlung im Einzelnen mit dem Arzt korrigiert, verbessert od. dgl. Eine automatisierte Überprüfung eines komplexeren Behandlungsplans, insbesondere auch im Hinblick auf etwaige Unverträglichkeiten, ist aber nicht vorgesehen und auch nicht möglich, da das Expertensystem überhaupt keinen Zugriff auf die Krankheitsgeschichte des Patienten, also auf seine Patientenakte, hat. Das mühselige Abfragen von bestimmten Randbedingungen zur Berücksichtigung beispielsweise solcher Unverträglichkeiten, wird aber durch das Expertensystem nach dieser US 5,517,405 allenfalls geführt, aber letztendlich nicht beschleunigt und vor allen Dingen ist für jeden Behandlungsplan und seine Überprüfung immer erneut ein Arzt erforderlich.

Der Erfindung liegt daher die Aufgabe zugrunde, ein System zu schaffen, das eine automatisierte Überprüfung von Behandlungsplänen ermöglicht.

Zur Lösung dieser Aufgabe ist ein solches System erfindungsgemäß gekennzeichnet durch ein Expertensystem mit implementierten Regeln, das unter Zugriff auf Patientendaten in unterschiedlichen Patientenakten und/oder bei unterschiedlichen Ärzten oder Kliniken selbstständig den Behandlungsplan einer Plausibilitätskontrolle unterzieht, insbesondere dass das Expertensystem den Behandlungsplan auf Kontraindikationen mit älteren Patientendaten überprüft.

Unter Behandlungsplan wird in diesem Kontext eine maschinenlesbare Darstellung einer zeitlichen Abfolge von Arbeitsschritten und Entscheidungen verstanden. Die Arbeitsschritte sind beschrieben durch eine allgemeine Definition, z. B. "Telemonitoring von Blutdruck" oder "Medikation mit .....", gegebenenfalls ergänzt durch für diesen Arbeitsschritt relevante patienten-spezifische Informationen, wie z. B. das Alter, das Gewicht, bekannte Arzneimittelunverträglichkeiten oder Multimorbidität etc. Mit diesem medizinischen Expertensystem wird die Konsistenz und Widerspruchsfreiheit eines in maschinenverständlicher Form abgespeicherten Behandlungsplans mit weiteren Informationsquellen geprüft, wie beispielsweise Datenbanken mit medizinischen Leitlinien und/oder Datenbanken mit medizinischem Lehrbuchwissen (Plausibilitätskontrolle).

Beispiele für solche Konsistenz-Prüfungen sind:
- Die Wahl der richtigen Medikation gemäß Alter oder medizinischer Vorgeschichte,
- Empfehlungen für körperliche Betätigung (physiotherapeutische Übungen etc.) gemäß Alter, körperlicher Rüstigkeit usw. des Patienten,
- Prüfung auf Unverträglichkeit oder Nebenwirkungen bei bekannter Zweit- oder Mehrfacherkrankung (Multimorbidität),
- Erkennen von in Guidelines über das zu behandelnde Krankheitsbild belegtem Wissen über nicht-effektive Maßnahmen oder
- Erkennen des Fehlens von Maßnahmen, die in Guidelines empfohlen werden.

Bei Erkennung einer Inkonsistenz, eines Widerspruchs oder gegebenenfalls auch einer Kontraindikation mit älteren Patientendaten, wird ein Warnhinweis an den Benutzer ausgegeben.

In Weiterbildung der Erfindung kann dabei vorgesehen sein, dass das System automatisch eine zyklische Wiederholung der Plausibilitätskontrolle unter Berücksichtigung geänderter Patientendaten oder Leitlinien durchführt, wobei die zyklische Wiederholung entweder in vorgebbaren Abständen erfolgen kann oder aber auch durch neue Dateneingaben, in die bei der Plausibilitätskontrolle verwendeten Datenbanken ausgelöst werden kann.

Das erfindungsgemäße System lässt sich dabei mit besonderem Vorteil in ein, in einer parallelen Patentanmeldung beschriebenes, System zu Erstellung von Behandlungsplänen integrieren, bei dem eine Datenbank mit einer Vielzahl von in digitaler Form gespeicherten Behandlungsmodulen vorgesehen ist, die mithilfe von Operatoren miteinander zu einem Behandlungsplan verknüpfbar sind, wobei bevorzugt eine graphische Benutzeroberfläche zur Unterstützung der Auswahl und der Zusammenstellung der vorgegebenen Behandlungsmodule sowie Eingabeeinrichtungen für Patientendaten zur Individualisierung des Behandlungsplans vorgesehen sein können.

Bei diesen aus übersichtlichen Makros oder Arbeitsmodulen aufgebauten Behandlungsplänen liegt bereits mit der Schaffung des Behandlungsplans eine maschinenlesbare Darstellung einer zeitlichen Abfolge von Arbeitsschritten und Entscheidungen vor, die im Sinne der vorliegenden Erfindung für die Überprüfung mithilfe eines Expertensystems notwendig und geeignet sind.

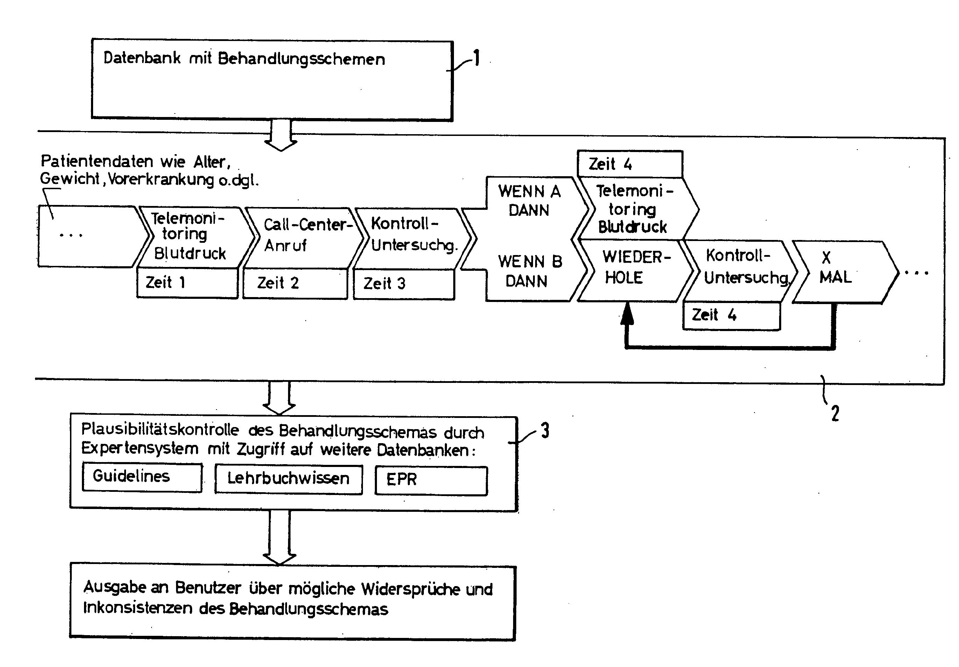

Weitere Vorteile, Merkmale und Einzelheiten der Erfindung ergeben sich aus der nachfolgenden Beschreibung eines Ausführungsbeispiels sowie anhand der Zeichnung, die ein schematisches Ablaufdiagramm des erfindungsgemäßen Systems zur Überprüfung von Behandlungsplänen darstellt.

Aus einer Datenbank 1 mit Behandlungsschemen und einzelnen Arbeitsmodulen zum Aufbau einer linearen oder verzweigen Modulkette gegebenenfalls mit Entscheidungsknoten und/oder Schleifen wird mithilfe einer nicht gezeigten grafischen Benutzeroberfläche ein aus standardisierten Arbeitsmodulen aufgebautes individuelles Behandlungsschema 2 für einen Patienten aufgebaut, wobei unter den Patientendaten in diesem individuellen Behandlungsschema beispielsweise die Angabe stehen soll, dass der Patient Diabetiker ist. Der in der Figur natürlich nur in einem kleinen Ausschnitt dargestellte Behandlungsplan enthält unter anderem ein Telemonitoring Blutdruck gefolgt von einem Call-Center-Anruf, um gegebenenfalls eine Kontrolluntersuchung durchzuführen, wobei dann ein Entscheidungsknoten entweder zu einem späterem Zeitpunkt 4 wiederum ein Telemonitoring Blutdruck veranlasst, oder aber bei anderem Ausgang der Kontrolluntersuchung, beispielsweise eine wiederholte Kontrolluntersuchung zur Zeit 4 veranlasst, die gegebenenfalls mehrfach wiederholt werden kann.

Im vorgegebenen Ausführungsbeispiel soll der Patient Diabetiker sein.

Das Expertensystem weiß aufgrund der implementierten Regeln, dass bei einem Diabetiker in jedem Fall eine Blutdruckmessung mit einer Blutzuckermessung kombiniert werden soll, da der Blutdruck allein ohne Kenntnis der Blutzuckers nicht die nötige Aussagekraft für weitere Entscheidungen und Beurteilungen bietet. Im vorgegebenen Behandlungsplan fehlt aber eine solche Blutzuckeruntersuchung und das Expertensystem, das gegebenenfalls Zugriff auf weitere Datenbanken zu Guidelines, Lehrbuchwissen oder einer elektronischen Patientenakte (EPR) besitzt, kann diesen möglichen Widerspruch oder Inkonsistenten des Behandlungsschemas erkennen und an den Benutzer melden.

Mithilfe des Expertensystems 3 können dabei auch Kontraindikationen mit erfasst werden.

## Patentansprüche

1. System zur Überprüfung von Behandlungsplänen, insbesondere im Zuge von Disease Management Services zur Patientenaufklärung, -weiterbildung und -motivation, gegebenenfalls kombiniert mit Telemonitoring kritischer Körperwerte und daraus abgeleiteter frühzeitiger Erkennung von Risikosituationen, **gekennzeichnet durch** ein Expertensystem mit in einer Computerdatenbank gespeicherten Expertenregeln, das unter Zugriff auf Patientendaten in unterschiedlichen Patientenakten und/oder bei unterschiedlichen Ärzten oder Kliniken selbstständig den Behandlungsplan einer Plausibilitätskontrolle unterzieht, insbesondere dass das Expertensystem den Behandlungsplan auf Kontraindikationen mit älteren Patientendaten überprüft.

2. System nach Anspruch 1, **dadurch gekennzeichnet, dass** der Behandlungsplan in Form einer maschinenlesbare Darstellung einer Abfolge von Arbeitsschritten und Entscheidungen aufgebaut ist.

3. System nach Anspruch 1 oder 2, **dadurch gekennzeichnet, dass** es mit weiteren Datenbanken zu medizinische Leitlinien, Lehrbuchwissen, EPR oder dergleichen verbunden ist.

4. System nach einem der Ansprüche 1 bis 3, **dadurch gekennzeichnet, dass** es automatisch eine zyklische, oder bei Eingang neuer Patientendaten oder medizinischen Leitlinien erfolgende, Wiederholung der Plausibilitätskontrolle unter Berücksichtigung geänderter Patientendaten oder Leitlinien durchführt.

5. System nach einem der Ansprüche 1 bis 4, **dadurch gekennzeichnet, dass** es in ein System zur Erstellung von Behandlungsplänen integriert ist, bei dem in einer Datenbank eine Vielzahl von Behandlungsmodulen in digitaler Form gespeichert sind, die mithilfe von Operatoren miteinander zu einem Behandlungsplan verknüpfbar sind, wobei bevorzugt eine graphische Benutzeroberfläche zur Unterstützung der Auswahl und der Zusammenstellung von vorgegebenen Behandlungsmodulen vorgesehen ist.
